# EUROPEAN PATENT SPECIFICATION

(11) **EP 4 173 564 B1**
(45) Date of publication and mention of the grant of the patent: **31.12.2025**
(21) Application number: 21845820.6
(22) Date of filing: 26.08.2021
(51) Int. Cl.: A61B 5/08, A61B 10/00, A61G 10/00, E04H 1/12

(54) **TESTING BOOTH FOR VIRUS SAMPLE COLLECTION HAVING EXAMINATION WINDOW ALLOWING CLOSE-CONTACT INSERTION OF SAMPLE COLLECTION TOOL**
PRÜFKABINE ZUR SAMMLUNG VON VIRUSPROBEN MIT EINEM PRÜFFENSTER ZUR ERMÖGLICHUNG DES EINSATZES EINES PROBENSAMMELWERKZEUGS MIT ENGEN KONTAKT
CABINE D'ESSAI POUR LA COLLECTE D'ÉCHANTILLONS DE VIRUS AYANT UNE FENÊTRE D'EXAMEN PERMETTANT L'INSERTION EN CONTACT ÉTROIT D'UN OUTIL DE COLLECTE D'ÉCHANTILLON

(43) Date of publication of application: 03.05.2023
(73) Proprietor: Park, Yong Nam, Gyeonggi-do 10915 (KR)
(72) Inventor: Park, Yong Nam, Gyeonggi-do 10915 (KR)
(74) Representative: Cabinet Chaillot
(86) International application number: PCT/KR2021/011474
(87) International publication number: WO 2022/019732

(56) References cited:
- CN-A- 111 166 391
- CN-A- 111 345 852
- CN-A- 113 081 591
- KR-A- 20120 055 294
- KR-A- 20180 093 679
- KR-A- 20180 093 679
- KR-A- 20190 043 350
- KR-B1- 100 983 626
- KR-B1- 102 178 101
- KR-B1- 102 247 756
- KR-Y1- 200 476 872

## Description

### [Technical Field]

The present invention relates to a test booth for collecting virus samples, wherein the test booth has an examination window allowing close-fitting insertion of sampling tools. More specifically, the present invention relates to a test booth for collecting virus samples, wherein the test booth has an examination window capable of increasing the efficiency of sample collection by a tester by excluding wearing of blunt rubber gloves and preventing contamination and cross-contamination through rubber gloves by sample collection swabs during a sample collection process.

### [Background Art]

Recently, the COVID-19 pandemic has resulted in numerous infections and deaths worldwide. In a situation where a medicine or vaccine is being developed, it is very important to wear a mask to prevent infection and to quickly find suspected infected persons.

As one of the quarantine activities for COVID-19, screening clinics are set up to collect samples from suspected infected persons, and medical staffs, who are testers, collect samples from suspected infected persons at the screening clinics.

In addition, in the process of collecting samples from suspected infected persons, preventing environmental contamination at sampling sites has emerged as an important issue to prevent transmission to medical staffs, who are testers, and the next subject.

To accurately perform a virus test, it is important for a medical staff to collect a sufficient amount of sample from the upper respiratory tract (oropharyngeal swab, nasopharyngeal swab, nasal swab, etc.).

When collecting a sample from the upper respiratory tract, a tester collects a sample from the oropharynx (mouth) and nasopharynx (nose) of a subject while the subject takes off a mask.

When collecting a sample from the mouth or nose, a tester must use sterile cotton swabs. Even when a subject is somewhat uncomfortable, it is necessary to collect a sufficient amount of sample by swabbing the posterior nasopharynx and palatopharyngeal wall several times.

During this sample collection process, a subject is likely to sneeze or cough due to stimulation of reflex nerves.

When a subject sneezes or coughs, ontamination may be spread to a wide space through droplets and aerosols at a sampling site.

Also, there is a high possibility that medical staffs, who are testers, will be infected. In practice, there are many cases in which medical staffs who examine suspected infected persons are infected.

Therefore, it is important to block contaminants such as droplets and aerosols discharged when a subject sneezes or breathes.

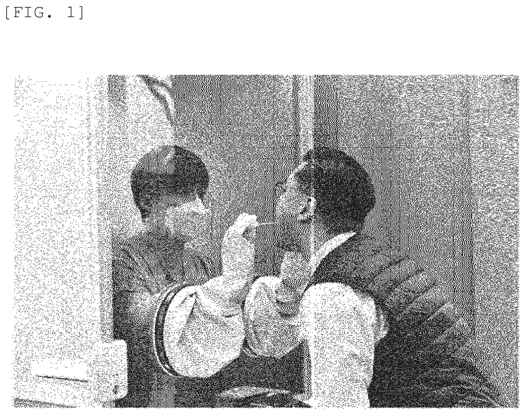

To solve this problem, as shown in FIG. 1, a test booth having a window wall installed between a tester and a subject is provided.

As a walking-thru test becomes possible by installing a test booth having a window wall, a tester can collect samples from a large number of people in a short period of time.

According to the walking-thru test, in a state where a medical staff is located in a booth and a subject is located outside the booth, the tester wears a rubber glove protruding out of the booth and collects a sample from the subject using a sample collection swab.

Due to the test booth having such a window wall, the tester does not need to wear a protective clothing because the tester is not affected by the outside
temperature, and thus the tester can collect samples in a comfortable environment.

In addition to ensuring the safety of a tester, when a test booth having a window wall is used, since the tester does not have to disinfect the inside of the test booth for each test, sample collection time may be reduced.

However, in the case of the test booth having the above described window wall, since a tester has to perform precise sample collection while wearing thick and blunt rubber gloves that prevent easy tearing, the tester cannot easily perform precise manipulations.

For example, due to wearing rubber gloves, it is not easy for a tester to hold the sanitary wrapping paper of sample collection swabs. In addition, after collecting a sample, it is difficult for the tester to easily perform a series of processes of cutting a sample collection swab and inserting the swab into a diagnostic tube.

Furthermore, while collecting a sample from the next subject while the contaminant of an infected person is on rubber gloves, the contaminant on the rubber gloves may come into contact with the sample collection swab of the next subject.

In this case, cross-contamination may occur, and test accuracy may be reduced. To solve these problems, when a tester wears disposable plastic gloves, it is difficult for the tester to completely exclude cross-contamination and perform precise manipulations. In addition, in the case of a large-scale test, test time may rather increase.

KR 102 247 756 B1 discloses a testing booth and is considered the closest prior art to the invention.

CN 113 081 591 A discloses a portable throat swab collection and isolation workbench. CN 111 345 852 A discloses a protective mask for swab collection sampling.

### [Non-Patent Document]

"Cheongsong-gun, introduction of 'Walking-thru' sampling" (Gyeongbuk Newspaper, April 12, 2020)

### [Disclosure]

### [Technical Problem]

Therefore, the present invention has been made in view of the above problems, and it is one object of the present invention to provide a test booth for collecting virus samples having an examination window capable of increasing the efficiency of sample collection work by enabling a tester to easily collect a sample by directly holding one end of a sample collection swab or the handle of a swab fitting portion into which the sample collection swab is inserted in a booth separated from the outside without a special quarantine device; and increasing the accuracy of a test by preventing contaminants from adhering to the sample collection swab.

### [Technical Solution]

In accordance with one aspect of the present invention, provided is a test booth for collecting virus samples , comprising a space portion from an outside in such a way as to loacate a tester therein and having a window wall facing a subject; and an examination window located on the window wall of the space portion in such a way to face the subject's face, characterised in that the examination window comprises: a swab entry/exit hole formed to pass through both sides of the examination window and having a given passage having a diameter that allows entry and exit of a sample collection swab; and a detachable cap detachably attached to the swab entry/exit hole formed on the space portion and having an open portion for guiding the sample collection swab to the passage of the swab entry/exit hole from the space portion, the open portion being open in one direction toward the passage of the swab entry/exit hole from the space portion.

The open portion may comprise incision lines formed on a given portion of the detachable cap, so that when the sample collection swab is inserted into the open portion, the open portion is incised by the external force generated by the insertion of the sample collection swab to form incised pieces, and the incised pieces are rolled into the detachable cap to allow air tightness of the detachable cap to be maintained.

In addition, an interference plate having a larger diameter than a diameter of the sample collection swab may be formed on the sample collection swab, wherein, when a tester in the space portion swabs a specific body part of a subject using the sample collection swab through the swab entry/exit holes and collects a sample, the interference plate is configured to detach the detachable caps from the swab entry/exit holes by interfering with the detachable caps so that the sample does not come into contact with the detachable caps.

In addition, a through hole connecting the space portion where a tester is located and an outside where a subject is located may be formed through the window wall, and the examination window may be configured to be attached to or detached from the through hole through the detachable means.

In addition, a connection portion made of a flexible material may be formed to penetrate an inside and outside of the examination window, a non-contact thermometer may be installed to be exposed to the outside of the examination window through the connection portion, and a handle of the non-contact thermometer may be installed to be exposed to the inside of the examination window through the connection portion.

In addition, the space portion is configured to be moved from a central control building through a moving means, and a wrinkled portion having elasticity may be installed between the space portion and the central control building.

### [Advantageous Effects]

A test booth for collecting virus samples having an examination window according tc the present invention has the following effects.

First, in a test booth for collecting virus samples having an examination window, a test can handle a sample collection swab with bare hands without wearing rubber gloves, allowing the tester to manipulate the sample collection swab precisely.

Accordingly, the test booth for collecting virus samples having an examination window has an effect of increasing sample collection efficiency by the tester.

Second, since the test booth for collecting virus samples having an examination window does not require rubber gloves, contaminants can be prevented from being transmitted to the next examiner's sample collection swab through rubber gloves.

Accordingly, the test booth for collecting virus samples having an examination window has an effect of increasing the safety of a test.

Third, since an examination window is configured to be detachable from the test booth for collecting virus samples having an examination window, the examination window can be replaced when necessary, and a hygienic sampling environment can be provided by separately sterilizing and disinfecting the examination window.

Fourth, since a moving means is installed in the test booth for collecting virus samples having an examination window, the test booth can be applied to the drive-thru method as well as the walking-thru method.

That is, when a tester approaches a subject in a vehicle, the test booth can move toward the subject in the vehicle.

Accordingly, the tester can conveniently and comfortably collect samples regardless of external temperature changes without being exposed to the outside.

### [Description of Drawings]

FIG. 1 is an image showing a situation in which a sample is collected from a subject through a test booth for collecting virus samples according to the prior art.
FIG. 2 is an exploded perspective view of a test booth for collecting virus samples having an examination window according to the first embodiment of the present invention.
FIG. 3 is a perspective view of a test booth for collecting virus samples having an examination window according to the first embodiment of the present invention.
FIG. 4 illustrates the cross-section of the main part of a test booth for collecting virus samples having an examination window according to the first embodiment of the present invention.
FIG. 5 illustrates a sample collection swab used in a test booth for collecting virus samples having an examination window according to the first embodiment of the present invention.
FIG. 6 is a perspective view of a detachable caps of a test booth for collecting virus samples having an examination window according to the first embodiment of the present invention.
FIGS. 7A and 7B illustrate a process of detaching a detachable caps of a test booth for collecting virus samples having an examination window according to the first embodiment of the present invention from an examination window toward a space portion.
FIG. 8 illustrates an examination window according to the first embodiment of the present invention into which a sample collection swab is inserted from the outside and a handle inside a booth.
FIG. 9 is a top view of a test booth for collecting virus samples having an examination window according to the second embodiment of the present invention that is configured to be movable using a moving means.
FIG. 10 is a side view showing the main part of a test booth for collecting virus samples having an examination window according to the second embodiment of the present invention that is configured to be movable using a moving means.
FIG. 11 shows an example of transporting a test booth for collecting virus samples having an examination window according to the third embodiment of the present invention using a trailer.

### [Best Mode]

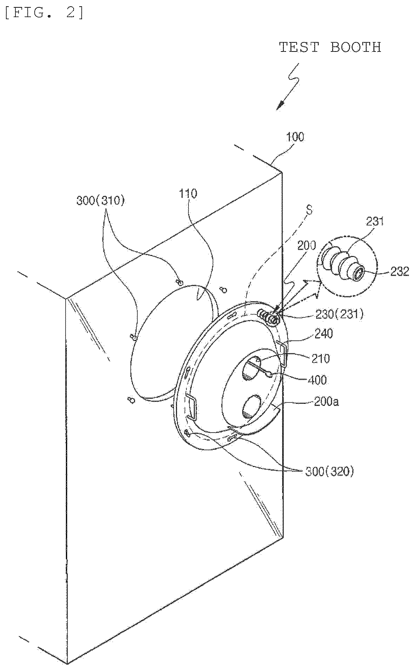

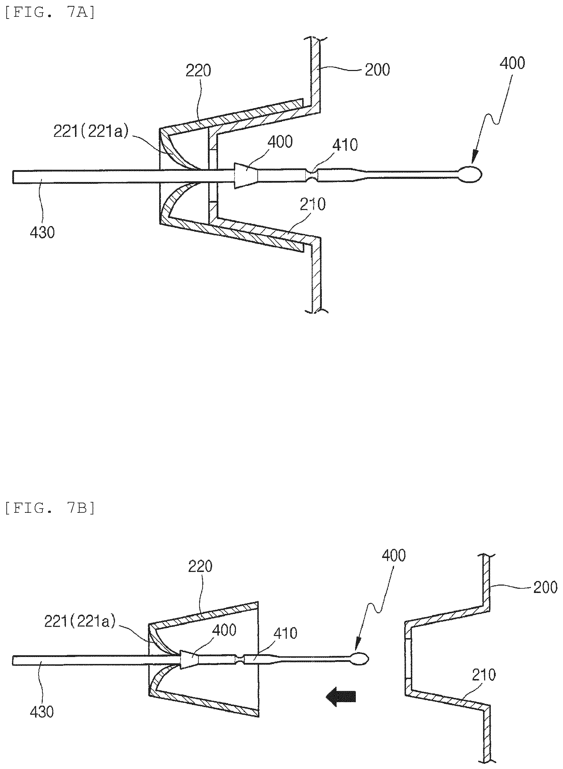

Hereinafter, referring to FIGS. 2 to 7B, a test booth for collecting virus samples having an examination window according to the first embodiment of the present invention will be described.

The test booth is configured so that a medical staff, who is a tester, may collect a sample from a subject outside the booth using a swab tool with bare hands inside the booth.

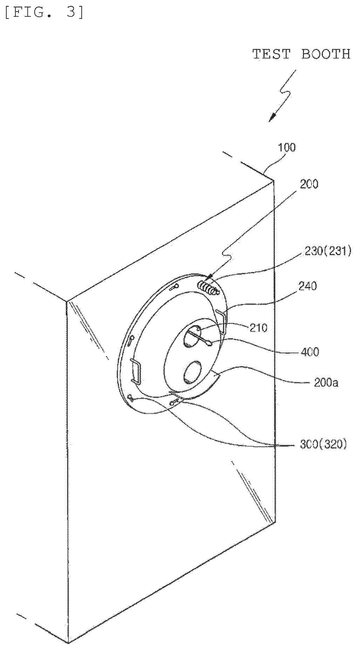

As shown in FIGS. 2 and 3, the test booth includes a space portion 100 and an examination window 200.

The space portion 100 provides a space in which a medical staff, who is a tester, is located, and is preferably formed to be transparent so that visibility outside the space portion 100 is secured.

The shape of the space portion 100 is not particularly limited, and may be any shape capable of providing a closed space from the outside.

As shown in FIG. 2, the space portion 100 is preferably provided in the form of a hexahedron. In this case, a through hole 110 through which the inside and outside of the space portion 100 communicate with each other is formed in the space portion 100. The through hole 110 is a space required for a tester to collect a sample from a subject through the examination window 200 to be described later, and is preferably formed in a circular shape.

In addition, detachable means 300 for attaching or detaching the examination window 200 are provided in the space portion 100, and attachment/detachment protrusions 310 are installed as the 12 detachable means 300 around the through hole 110.

In this case, the diameter of the end of the attachment/detachment protrusion 310 is larger than that of the attachment/detachment protrusion 310.

The examination window 200 provides a passage through which a tester pushes a sample collection swab 400 from the inside of the space portion 100 to the outside of the space portion 100, and is made of a transparent material.

The examination window 200 is preferably formed in a circular shape suitable for covering the through hole 110, and is configured to be attached tc or detached from the space portion 100 through the detachable means 300.

For this configuration, attachment/detachment holes 320 corresponding to the attachment/detachment protrusions 310 of the detachable means 300 are formed along the circumference of the examination window 200.

The attachment/detachment holes 320 are preferably formed in the form of a long hole, and the diameter of the end of the attachment/detachment hole 320 is larger than that of the attachment/detachment hole 320.

After the attachment/detachment protrusions 310 are inserted through the ends of the attachment/detachment holes 320, the examination window 200 may be coupled to the space portion by rotating the examination window 200 so that the ends of the attachment/detachment protrusions 310 are caught in the attachment/detachment holes 320.

As described above, the examination window 200 may be configured to be detachable from the space portion 100 through the detachable means 300.

Alternatively, the examination window 200 may be integrally formed in the space portion 100.

As shown in FIG. 2, a support portion 200a for supporting the subject's chin is preferably formed below the examination window 200.

The support portion 200a is preferably formed in an arc shape along the curvature of the examination window 200.

Although not shown, extension pieces may be extended to opposite sides of the support portion 200a to support opposite cheeks of a subject.

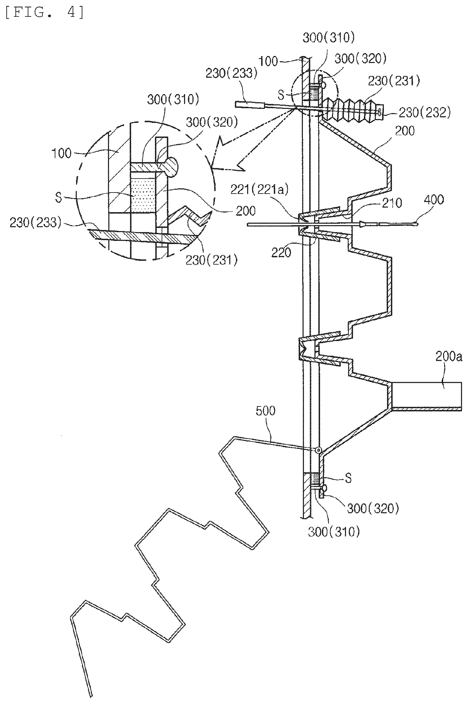

As shown in FIG. 4, the examination window 200 includes swab entry/exit holes 210 and detachable caps 220.

The swab entry/exit holes 210 provide a passage through which the sample collection swab 400 enters and exits the subject's respiratory system, and are formed at the center of the examination window 200.

The number of the swab entry/exit holes 210 is preferably one or more.

Considering that the sample collection swab 400 is usually inserted into the nose or mouth of a subject, the number of the swab entry/exit holes 210 is preferably two, as shown in FIGS. 2 to 4.

The swab entry/exit holes 210 protrude toward the inner side of the examination window 200, i.e., toward the space portion 100, and are formed to be opened and closed through the detachable caps 220.

The swab entry/exit holes 21C are preferably formed to be inclined so that the detachable caps 220 are fitted into the swab entry/exit holes 210.

That is, the swab entry/exit holes 210 are formed to protrude from the examination window 200 toward the space portion 100, and the diameter of the swab entry/exit holes 210 gradually decreases toward the space portion 100.

This configuration of the swab entry/exit holes 210 may be effective for press-fitting of the detachable caps 220.

In addition, as shown in FIGS. 2 and 4, a sealing member (S) may be installed on the circumferential surface of the examination window 200 to maintain air tightness with the space portion.

The detachable caps 220 of the examination window 200 are configured to cover the swab entry/exit holes 210 to close the passage of the swab entry/exit holes 210 and configured to be attached to or detached from the swab entry/exit holes 210.

The detachable caps 220 are preferably composed of a resin material so that the detachable caps 220 are forcibly fitted into the swab entry/exit holes 210.

As shown in FIGS. 4, 7A, and 7B, the detachable caps 220 are preferably formed to have an inclined surface.

The detachable caps 220 are formed in a hollow shape with one side opened, and an opening means 221 is formed on the other side of the detachable caps 220.

The opening means 221 is a component for guiding insertion of the sample collection swab 400 into the swab entry/exit holes 210, and are configured to be opened only in one direction toward the swab entry/exit holes 210.

With this configuration, the detachable caps 220 may maintain air tightness even when the sample collection swab 400 is inserted into the detachable caps 220 through the opening means 221.

The configuration of the opening means 221 is not particularly limited.

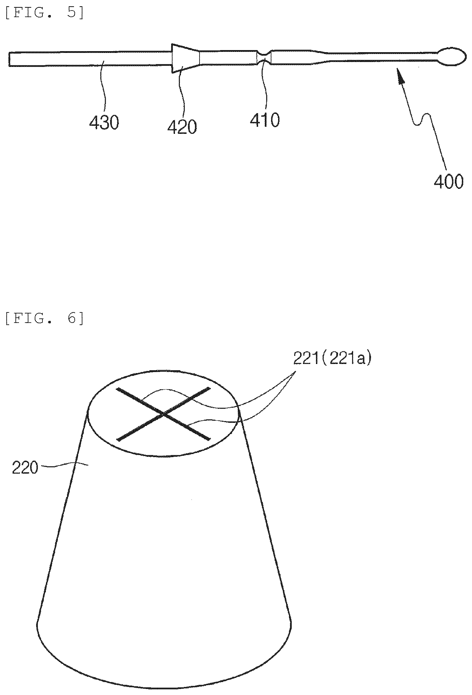

As shown in FIG. 6, the opening means 221 is preferably formed as an incision line capable of incising the other side of the detachable caps 220 in all directions.

The thickness of an incision line 221 is smaller than the thickness of the detachable caps 220, and the incision line 221 is configured to be incised when external force is applied using the sample collection swab 400.

Accordingly, as shown in FIG. 7A, due to the material characteristics of the detachable caps 220, as soon as the sample collection swab 400 passes, the opening means 221 is cut toward the swab entry/exit holes 210 and spreads in one direction to form incision pieces 221a.

Alternatively, the incision line 221may be provided in an already incised state. In addition, although not shown, the incision line 221 may be provided in various forms.

For example, the incision line 221 may be formed in a circular shape with only one side fixed.

In this case, when force is applied with the sample collection swab 400, the incision line 221 may be configured so that a circular incision piece is rolled around one side.

In addition, the sample collection swab 400 configured to pass through the examination window 200 through the swab entry/exit holes 210 will be described.

As shown in FIG. 5, 16 the sample collection swab 400 includes a cutting portion 410, an interference plate 420, and a handle 430.

The cutting portion 410 is a portion for separating the sample collection swab 400 and the handle 430, and is formed so that the diameter thereof is smaller than the diameters of the sample collection swab 400 and the handle 430.

When a tester in the space portion 100 swabs a specific body part of a subject using the sample collection swab 400 through the swab entry/exit holes 210 and collects a sample, the interference plate 420 is configured to detach the detachable caps 220 from the swab entry/exit holes 210 by interfering with the detachable caps 220 so that the sample does not come into contact with the detachable caps 220.

The interference plate 420 is formed so that the diameter thereof is greater than the diameter of the handle 430 and smaller than the diameter of the passage of the swab entry/exit holes 210.

The handle 430 has a bar shape suitable for a tester to grip. In addition, although not shown, the interference plate 420 may be composed of concave-convex wrinkles.

That is, the sample collection swab 400 may be configured to remove the detachable caps 220 through the wrinkled portion.

The configuration of the wrinkled portion may be a configuration corresponding to the circular incision pieces of the detachable caps 220 described above.

In addition, as shown in FIG. 2, the examination window 200 preferably further includes a non-contact thermometer 230 and grip portions 240.

The non-contact thermometer 230 is a component for measuring the subject's body temperature, and is installed in the examination window 200 through a connection portion 231 made of a flexible material.

That is, the non-contact thermometer 230 is provided so that a tester detects heat without directly facing a subject.

Thus, the present invention enables complete non-face-to-face contact in a series of processes for collecting a sample from a subject.

The non-contact thermometer 230 includes a sensor 232, and the sensor 232 is exposed to the outside of the examination window 200.

In this case, as shown in FIG. 4, a handle 233 of the non-contact thermometer 230 is positioned inside the examination window 200 so that a tester may adjust the position of the sensor 232. The grip portions 240 are configured so that a manager easily carries the examination window 200, and is installed on opposite sides of the examination window 200.

Hereinafter, a process of collecting a sample from a subject by a tester in a test booth for collecting virus samples having an examination window configured as described above will be described.

A test booth is installed adjacent to a central control building, and samples are collected from suspected infected persons passing through the test booth according to the walking-thru method.

At this time, a tester is located in the 18 test booth, and a subject is positioned so that the subject's face faces the examination window 200 of the test booth.

At this time, the subject puts his/her chin on the supporting portion 200a and waits for examination.

When management of sample collection swabs is to be performed only inside a test booth, an examination window that allows sample collection swabs to be inserted inside the test booth is used.

At this time, a tester in the test booth pushes the sample collection swab 400 into the opening means 221 of the detachable cap 220 and inserts the sample collection swab 400 into the passage of the swab entry/exit hole 210.

At this time, as shown in FIG. 7A, the sample collection swab 400 is inserted while incising and widening the incision line 221, which is an opening means, and then the sample collection swab 400 is inserted into the subject's respiratory tract through the passage of the swab entry/exit hole 210.

In this process, the open incision pieces 221a are restored and adhere to the handle 430 of the sample collection swab 400. Accordingly, the inside of the examination window 200, i.e., the inside of the space portion 100 may be maintain an enclosed state.

Even when the subject sneezes toward the examination window 200, since the incision pieces 221a do not spread outward, the passage of the swab entry/exit hole 210 may remain airtight.

Accordingly, the subject's breath or droplets are prevented from entering the space portion 100 where the tester is located.

Then, the tester checks the subject's face through the examination window 200 and performs swabbing using the sample collection swab 400.

At this time, since the tester handles the sample collection swab 400 with bare hands inside the space portion 100, the efficiency of the sample collection operation may be increased. After swabbing, the tester pulls out the sample collection swab 400 toward the inside of the examination window 200, i.e., the space portion 100.

At this time, when the tester pulls the sample collection swab 400 into the examination window 200, as shown in FIG. 7A, the sample collection swab 400 is moved to the left in the drawing, and then the interference plate 420 interferes with the incision pieces 221a to apply pressure to the detachable cap 220.

Then, as shown in FIG. 7A, the sample collection swab 400 separates the detachable cap 220 from the swab entry/exit hole 210.

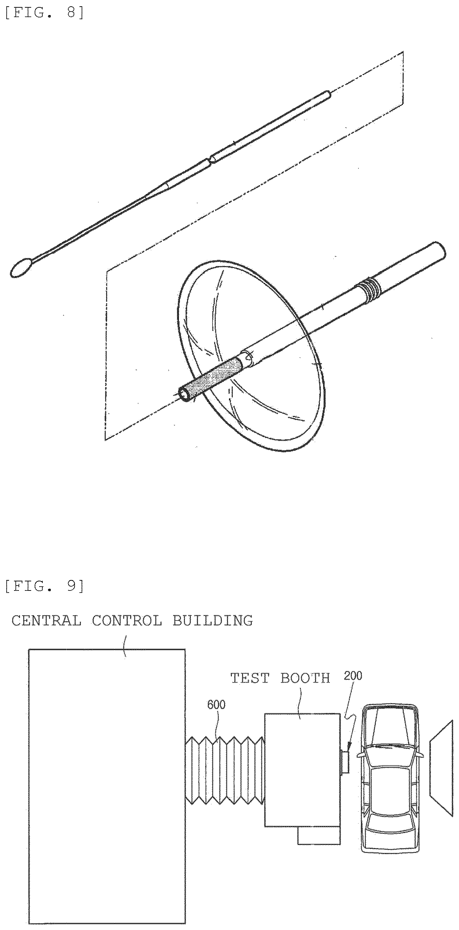

When management of sample collection swabs is to be performed outside an examination booth, as shown in FIG. 8, an examination window having a swab fitting portion into which a sample collection swab is inserted outside the examination booth is used.

A tester collects a sample by holding the fitting portion and handles protruding into the booth and connected to the examination window.

Thereafter, the tester cuts the cutting portion 410 of the sample collection swab 400 and performs virus diagnosis using a test kit.

At this time, since the process of cutting the sample collection swab 400 may also be performed by the tester with bare hands, the cutting process may be performed accurately and quickly.

In addition, when the process of collecting a sample from a subject is completed, as show in FIG. 7B, the detachable cap 220 is separated and the swab entry/exit hole 210 is opened.

After sampling, the subject's breath or ambient aerosol may flow in through the swab entry/exit hole 210.

When the subject sneezes, droplets may flow into the examination window 200 through the swab entry/exit hole 210.

To prevent contaminants from entering the examination window 200 from the subject after sample collection is completed, as shown in FIG. 4, the swab entry/exit hole 210 is shielded with a shielding cover 500 provided inside the examination window 200.

The shielding cover 500 is shaft-coupled to the inner lower part of the examination window 200, and is provided so as to rotate toward the swab entry/exit hole 210 around the shaft.

In this case, although not shown, a fastening means (not shown) may be installed at the end of the shielding cover 500 so that the shielding cover 500 is rotated toward the inside of the examination window 200 to shield the swab entry/exit hole 210, and then movement of the shielding cover 500 is constrained.

As described above, the test booth is provided as a structure on one side of the central control building, and a walk-thru method in which a sample is collected while a subject passes through the test booth may be used.

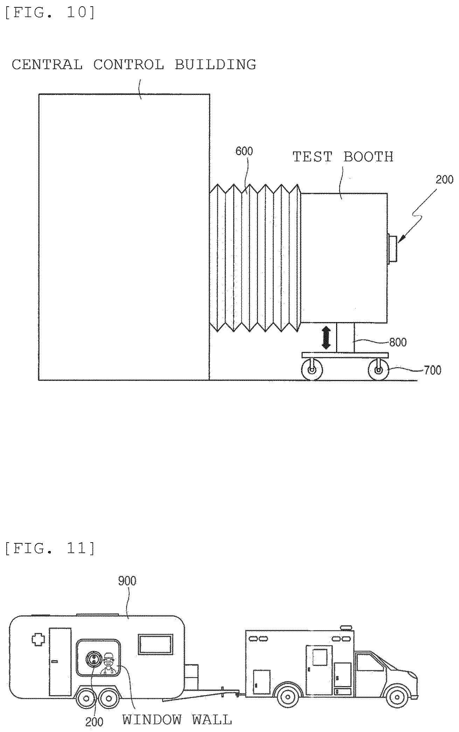

In addition, a drive-thru method may be used by allowing the test booth to move and access a subject in a vehicle. This method is presented as a second embodiment of the present invention, and will be described with reference to FIGS. 9 and 10.

Prior to the description, the same components as those in the first embodiment are indicated by reference numerals.

A test booth is provided as a separate body from a central control building, and a wrinkled portion 600 capable of performing stretching action is installed between the test booth and the central control building.

The wrinkled portion 600 facilitates movement and access of the test booth based on the central control building while performing stretching action.

The wrinkled portion 600 provides a connection passage connecting the central control building and the test booth.

The configuration of the wrinkled portion 600 is similar to that of a boarding bridge that connects an airport boarding gate and an airplane to provide a passage.

In addition, as shown in FIG. 10, in the test booth, a driving means 700 for moving the test booth is installed and a height adjusting means 800 for adjusting the height of the test booth is installed.

The driving means 700 includes a driving unit including a driving motor and wheels capable of performing a rolling action through the power of the driving unit.

In this case, a steering device capable of changing the direction of the wheels may also be configured.

The height adjusting means 800 allows the position of the test booth to correspond to the driver's seat position of a vehicle when the drive-thru test method is performed.

Since the height of the driver's seat is different for each type of vehicle, the height adjusting means 800 is configured to elevate the test booth.

The height adjusting means 800 may include a hydraulic cylinder and the like. Any apparatus capable of generating power capable of elevating the test booth may be used as the height adjusting means 800.

In addition, the test booth may be connected to a mobile trailer and transported to a location where group testing is required.

This case is presented as a third embodiment of the present invention and described with reference to FIG. 11.

As shown in FIG. 11, an ambulance and a trailer 900 are provided, and the examination window 200 is installed on one side of the trailer 900.

A general automobile may be used instead of an ambulance, and the examination window 200 has the same configuration as the examination window 200 described in the first embodiment. In this way, when the test booth is connected to the trailer 900, since the tester may directly visit a subject regardless of distance, a virus test may be performed quickly.

As described above, the test booth for collecting virus samples having an examination window according to the present invention is configured so that a tester may collect a sample from a subject outside the test booth while handling a sample collection swab tool without wearing rubber gloves while being located inside the test booth.

Accordingly, the present invention may provide a comfortable test environment to testers while securing infection safety of testers, and may be applied to various test methods such as walk-thru and drivethru.

Hereinafter, specific examples of the present invention have been described in detail. However, these examples are provided for illustrative purposes only and should not be construed as limiting the scope of the present invention.

### [Description of Symbols]

100: SPACE PORTION
110: THROUGH HOLE
200: EXAMINATION WINDOW
200A: SUPPORT PORTION
210: SWAB ENTRY/EXIT HOLES
220: DETACHABLE CAPS
221: OPENING MEANS (INCISION LINE)
221A: INCISION PIECES
230: NON-CONTACT THERMOMETER
231: CONNECTION PORTION
232: SENSOR 233: HANDLE
240: GRIP PORTIONS
300: DETACHABLE MEANS
310: ATTACHMENT/DETACHMENT PROTRUSIONS
320: ATTACHMENT/DETACHMENT HOLES
400: SAMPLE COLLECTION SWAB
410: CUTTING PORTION
420: INTERFERENCE PLATE
430: HANDLE
500: SHIELDING COVER
600: WRINKLED PORTION
700: DRIVING MEANS
800: HEIGHT ADJUSTING MEANS
900: TRAILER

## Claims

1. A test booth for collecting virus samples , comprising a space portion (100) blocked from an outside in such a way as to locate a tester therein and having a window wall facing a subject; and an examination window (200) located on the window wall of the space portion (100) in such a way to face the subject's face, **characterised in that** the examination window (200) comprises:
a swab entry/exit hole (210) formed to pass through both sides of the examination window (200) and having a given passage having a diameter that allows entry and exit of a sample collection swab (400); and
a detachable cap (220) detachably attached to the swab entry/exit hole (210) formed on the space portion (100) side and having an open portion (221) for guiding the sample collection swab (400) to the passage of the swab entry/exit hole (210) from the space portion (100), the open portion (221) being open in one direction toward the passage of the swab entry/exit hole (210) from the space portion (100).

2. The test booth according to claim 1, wherein the open portion (221) comprises incision lines formed on a given portion of the detachable cap (220), so that when the sample collection swab (400) is inserted into the open portion (221), the open portion (221) is incised by the external force generated by the insertion of the sample collection swab to form incised pieces (221a), and the incised pieces (221a) are rolled into the detachable cap (220) to allow air tightness of the detachable cap (220) to be maintained.

3. The test booth according to claim 1 or 2, wherein an interference plate (420) having a larger diameter than a diameter of the sample collection swab (400) is formed on the sample collection swab (400), wherein, when a tester in the space portion (100) swabs a specific body part of a subject using the sample collection swab (400) through the swab entry/exit hole (210) and collects a sample, the interference plate (420) is configured to detach the detachable cap (220) from the swab entry/exit hole (210) by interfering with the detachable cap (220) so that the sample does not come into contact with the detachable cap (220).

4. The test booth according to claim 1 or 2, wherein a through hole (110) connecting the space portion (100) where a tester is located and an outside where a subject is located is formed through the window wall, and the examination window (200) is configured to be attached to or detached from the through hole (110) through detachable means (300).

5. The test booth according to claim 1 or 2, wherein a connection portion (231) made of a flexible material is formed to penetrate an inside and outside of the examination window (200), a non-contact thermometer (230) is installed to be exposed to the outside of the examination window (200) through the connection portion (231), and a handle (233) of the non-contact thermometer (230) is installed to be exposed to the inside of the examination window (200) through the connection portion (231).

6. The test booth according to claim 1 or 2, wherein the space portion (100) is configured to be moved from a central control building through a moving means (700), and a wrinkled portion (600) having elasticity is installed between the space portion (100) and the central control building.

## Patentansprüche

1. - Prüfkabine zur Sammlung von Virusproben, umfassend einen Raumabschnitt (100), der von einem Außenbereich derart abgetrennt ist, dass sich ein Prüfer darin befinden kann, und umfassend eine Fensterwand, die einem Subjekt zugewandt ist; und ein Prüffenster (200), das sich auf der Fensterwand des Raumabschnitts (100) derart befindet, dass es dem Gesicht des Subjekts zugewandt ist,
**dadurch gekennzeichnet, dass** das Prüffenster (200) Folgendes umfasst:
eine Tupfer-Eintritts-/Austrittsöffnung (210), die gebildet ist, um durch beide Seiten des Prüffensters (200) zu verlaufen, und einen bestimmten Durchgang aufweist, der einen Durchmesser aufweist, der den Eintritt und Austritt eines Probesammeltupfers (400) ermöglicht; und
eine abnehmbare Kappe (220), die abnehmbar an die Tupfer-Eintritts-/Austrittsöffnung (210) befestigt ist, die an der Seite des Raumabschnitts (100) gebildet ist und einen offenen Abschnitt (221) zum Führen des Probesammeltupfers (400) zum Durchgang der Tupfer-Eintritts-/Austrittsöffnung (210) vom Raumabschnitt (100) aufweist, wobei der offene Abschnitt (221) in einer Richtung hin zum Durchgang der Tupfer-Eintritts-/Austrittsöffnung (210) vom Raumabschnitt (100) offen ist.

2. - Prüfkabine nach Anspruch 1, wobei der offene Abschnitt (221) Schnittlinien umfasst, die auf einem bestimmten Abschnitt der abnehmbaren Kappe (220) gebildet sind, sodass, wenn der Probensammeltupfer (400) in den offenen Abschnitt (221) eingeführt wird, der offene Abschnitt (221) durch die äußere Kraft eingeschnitten wird, die durch das Einführen des Probesammeltupfers erzeugt wird, um eingeschnittene Stücke (221a) zu bilden, und die eingeschnitten Stücke (221a) werden in die abnehmbare Kappe (220) eingerollt, um zu ermöglichen, dass die Luftdichtigkeit der abnehmbaren Kappe (220) beibehalten wird.

3. - Prüfkabine nach Anspruch 1 oder 2, wobei eine Interferenzplatte (420) mit einem größeren Durchmesser als einem Durchmesser des Probesammeltupfers (400) auf dem Probesammeltupfer (400) gebildet ist, wobei, wenn ein Prüfer im Raumabschnitt (100) einen bestimmten Körperteil eines Subjekts unter Verwendung des Probensammeltupfers (400) durch die Tupfer-Eintritts-/Austrittsöffnung (210) abtupft und eine Probe sammelt, die Interferenzplatte (420) konfiguriert ist, um die abnehmbare Kappe (220) von der Tupfer-Eintritts-/Austrittsöffnung (210) durch Interferieren mit der abnehmbaren Kappe (220) abzunehmen, sodass die Probe nicht mit der abnehmbaren Kappe (220) in Kontakt kommt.

4. - Prüfkabine nach Anspruch 1 oder 2, wobei eine Durchgangsöffnung (110), die den Raumabschnitt (100), in dem sich ein Prüfer befindet, und einen Außenbereich, in dem sich ein Subjekt befindet, verbindet, durch die Fensterwand gebildet ist, und das Prüffenster (200) konfiguriert ist, um durch abnehmbare Mittel (300) an die Durchgangsöffnung (110) befestigt oder von dieser abgenommen zu sein.

5. - Prüfkabine nach Anspruch 1 oder 2, wobei ein Verbindungabschnitt (231), hergestellt aus einem flexiblen Material, gebildet ist, um in eine Innenseite und Außenseite des Prüffensters (200) einzudringen, ein kontaktloses Thermometer (230) installiert ist, um durch den Verbindungsabschnitt (231) an die Außenseite des Prüffensters (200) ausgesetzt zu sein, und ein Griff (233) des kontaktlosen Thermometers (230) installiert ist, um durch den Verbindungsabschnitt (231) an die Innenseite des Prüffensters (200) ausgesetzt zu sein.

6. - Prüfkabine nach Anspruch 1 oder 2, wobei der Raumabschnitt (100) konfiguriert ist, um von einem zentralen Kontrollgebäude mit Hilfe eines Bewegungsmittels (700) bewegt zu werden, und ein faltiger Abschnitt (600) mit Elastizität zwischen dem Raumabschnitt (100) und dem zentralen Kontrollgebäude installiert ist.

## Revendications

1. - Cabine de test pour la collecte d'échantillons de virus, comprenant une partie espace (100) bloquée depuis l'extérieur de manière à y placer un testeur et ayant une paroi vitrée faisant face à un sujet ; et une fenêtre d'examen (200) située sur la paroi vitrée de la partie espace (100) de manière à faire face au visage du sujet, **caractérisée par le fait que** la fenêtre d'examen (200) comprend :
un trou d'entrée/sortie d'écouvillon (210) formé pour traverser les deux côtés de la fenêtre d'examen (200) et ayant un passage donné d'un diamètre permettant l'entrée et la sortie d'un écouvillon de collecte d'échantillon (400) ; et
un capuchon détachable (220) fixé de manière détachable au trou d'entrée/sortie d'écouvillon (210) formé du côté de la partie espace (100) et ayant une partie ouverte (221) pour guider l'écouvillon de collecte d'échantillon (400) jusqu'au passage du trou d'entrée/sortie d'écouvillon (210) à partir de la partie espace (100), la partie ouverte (221) étant ouverte dans une direction vers le passage du trou d'entrée/sortie d'écouvillon (210) à partir de la partie espace (100).

2. - Cabine de test selon la revendication 1, dans laquelle la partie ouverte (221) comprend des lignes d'incision formées sur une partie donnée du capuchon détachable (220), de telle sorte que lorsque l'écouvillon de collecte d'échantillon (400) est introduit dans la partie ouverte (221), la partie ouverte (221) est incisée par la force externe générée par l'introduction de l'écouvillon de collecte d'échantillon pour former des pièces incisées (221a), et les pièces incisées (221a) sont roulées dans le capuchon détachable (220) pour permettre le maintien de l'étanchéité à l'air du capuchon détachable (220).

3. - Cabine de test selon la revendication 1 ou 2, dans laquelle une plaque d'interférence (420) ayant un diamètre plus grand que le diamètre de l'écouvillon de collecte d'échantillon (400) est formée sur l'écouvillon de collecte d'échantillon (400), où, lorsqu'un testeur dans la partie espace (100) écouvillonne une partie de corps spécifique d'un sujet à l'aide de l'écouvillon de collecte d'échantillon (400) à travers le trou d'entrée/sortie d'écouvillon (210) et collecte un échantillon, la plaque d'interférence (420) est configurée pour détacher le capuchon détachable (220) à partir du trou d'entrée/sortie d'écouvillon (210) par interférence avec le capuchon détachable (220) de telle sorte que l'échantillon n'entre pas en contact avec le capuchon détachable (220).

4. - Cabine de test selon la revendication 1 ou 2, dans laquelle un trou traversant (110) reliant la partie espace (100) où se trouve un testeur et un extérieur où se trouve un sujet est formé à travers la paroi vitrée, et la fenêtre d'examen (200) est configurée pour être attachée au ou détachée du trou traversant (110) par un moyen détachable (300).

5. - Cabine de test selon la revendication 1 ou 2, dans laquelle une partie de liaison (231) faite d'un matériau flexible est formée pour pénétrer à l'intérieur et à l'extérieur de la fenêtre d'examen (200), un thermomètre sans contact (230) est installé pour être exposé à l'extérieur de la fenêtre d'examen (200) par la partie de liaison (231), et une poignée (233) du thermomètre sans contact (230) est installée pour être exposée à l'intérieur de la fenêtre d'examen (200) par la partie de liaison (231).

6. - Cabine de test selon la revendication 1 ou 2, dans laquelle la partie espace (100) est configurée pour être déplacée à partir d'un bâtiment de contrôle central par un moyen de déplacement (700), et une partie en accordéon (600) ayant une élasticité est installée entre la partie espace (100) et le bâtiment de contrôle central.
